# EUROPÄISCHE PATENTANMELDUNG

(11) **EP 1 873 198 A1**
(43) Veröffentlichungstag der Anmeldung: **02.01.2008**
(21) Anmeldenummer: 07109325.6
(22) Anmeldetag: 31.05.2007
(51) Int. Cl.: C08K 5/10, C08K 5/11, C08K 5/12, C08L 27/06

(54) **Mischungen aus Alkylester und Benzylester von Polycarbonsäuren**

(30) Priorität: 30.06.2006 DE 102006030306; 14.07.2006 DE 102006033097
(71) Anmelder: LANXESS Deutschland GmbH, 51369 Leverkusen (DE)
(72) Erfinder: Weiß, Thomas, 40789, Monheim (DE); Kuckert, Eberhard, 53175, Leverkusen (DE); Wiedemeier, Melanie, 41540, Dormagen (DE); Hansel, Jan-Gerd, 51069, Köln (DE)

(57) **Zusammenfassung**

Estennischungen aus Polyalkylestem aromatischer oder aliphatischer Polycarbonsäuren, Alkylbenzylestern aromatischer oder aliphatischer Polycarbonsäuren und Polybenzylestem aromatischer oder aliphatischer Polycarbonsäuren, sowie ein chemisches Verfahren zur Herstellung eines Teils dieser Estermischungen und ihre Verwendung als Weichmacher.

## Beschreibung

Die Erfindung betrifft Estermischungen aus Polyalkylestern aromatischer oder aliphatischer Polycarbonsäuren, Alkylbenzylestern aromatischer oder aliphatischer Polycarbonsäuren und Polybenzylestern aromatischer oder aliphatischer Polycarbonsäuren, sowie ein chemisches Verfahren zur Herstellung eines Teils dieser Estermischungen und ihre Verwendung als Weichmacher.

Zur Verarbeitung von Kunststoffen wie Polyvinylchlorid werden seit Jahrzehnten Weichmacher eingesetzt. In letzter Zeit steigen die Anforderungen an die Weichmacher im Hinblick auf Leistungsfähigkeit und Unbedenklichkeit gegenüber Mensch und Umwelt. Eine wichtige Forderung betrifft eine möglichst niedrige Lösetemperatur. Unter der Lösetemperatur wird im Zusammenhang mit Weichmachern die Temperatur verstanden, bei der aus einer Polyvinylchlorid-Dispersion in einem Weichmacher eine homogene Phase wird (L. Meier: "Weichmacher", in R. Gächter, H. Müller (Ed.): Taschenbuch der Kunststoffadditive, 3. Ausgabe, S. 361 - S. 362, Hanser Verlag, München 1990). Weichmacher mit niedriger Lösetemperatur ermöglichen eine schnelle und energiesparende Verarbeitung.

In Anwendungen, in denen eine niedrige Lösetemperatur gefordert ist, werden nach dem Stand der Technik überwiegend Alkylbenzylester von aromatischen Polycarbonsäuren, wie beispielsweise das Butylbenzylphthalat, eingesetzt (L. Meier: "Weichmacher", in R. Gächter, H. Müller (Ed.): Taschenbuch der Kunststoffadditive, 3. Ausgabe, S. 397, Hanser Verlag, München 1990). Diese sind kostengünstig durch Umsetzung von aliphatischen Alkoholen mit aromatischen Polycarbonsäuren oder ihren cyclischen Anhydriden und Benzylhalogeniden wie zum Beispiel Benzylchlorid in Gegenwart einer Base herstellbar. Das Herstellverfahren kann schrittweise oder in einem Eintopfverfahren (DE-A 1 468 373 für Benzylalkylphthalate, DE-A 1 593 047 für Benzylalkyltrimellitate) durchgeführt werden.

Neben einer niedrigen Lösetemperatur wird von zeitgemäßen Weichmachern aber auch eine niedrige Flüchtigkeit verlangt. Die Flüchtigkeit von Weichmachern führt zu unerwünschter Versprödung des Weich-Polyvinylchlorid sowie zur Belastung mit sogenannten Flüchtigen Organischen Substanzen (VOC), die in verbrauchernahen Anwendungen vermieden werden soll.

Butylbenzylphthalat weist eine sehr niedrige Lösetemperatur auf, ist allerdings flüchtig. Aus GB 969,911 sind Alkylbenzylphthalate mit längerer Alkylkette, wie zum Beispiel Benzyloctylphthalat, bekannt, die weniger flüchtig sind. Nachteilig bei allen Alkylbenzylphthalaten ist allerdings der Anfall großer Mengen korrosiver Salze bei ihrer Herstellung durch Benzylierung mit Benzylchlorid. Dies erfordert eine wässrige Aufarbeitung mit Anfall kostenpflichtigen Abwasser s. Weiterhin erfordert die korrosive Wirkung des bei hoher Temperatur HCl-abspaltenden Benzylchlorids die Verwendung korrosionsresistenter Apparaturen.

Die Herstellung von Butylbenzylphthalat durch Veresterung einer Mischung aus Phthalsäureanhydrid, n-Butanol und Benzylalkohol wird in L. M. Bolotina, E. G. Maksimenko, G.V. Maksimova, Khimicheskaya Promyshlennost (Moskau) 1978, 54 (4), 257 - 259 (Chemical Abstracts 1978:423904) beschrieben.

Die Aufgabe der vorliegenden Erfindung bestand darin, Weichmacher mit niedriger Lösetemperatur und niedriger Flüchtigkeit zu finden, die einfach, ohne Abwasserbildung und ohne Benzylchlorid herstellbar sind.

Überraschend wurde nun gefunden, dass Estermischungen aus Polyalkylestern aromatischer oder aliphatischer Polycarbonsäuren oder Alkylbenzylestern aromatischer oder aliphatischer Polycarbonsäuren oder Polybenzylestern aromatischer oder aliphatischer Polycarbonsäuren unerwartet niedrige Lösetemperaturen aufweisen und dabei eine niedrige Flüchtigkeit besitzen. Gegenstand dieser Erfindung sind deshalb Estermischungen der folgenden Zusammensetzung:
a) 2-98 Gew.-% eines oder mehrerer Ester der Struktur Z-(COOR²)ₘ₋ₙ(COOR¹)ₙ und
b) 2-98 Gew.-% eines Esters der Struktur Z-(COOR²)ₘ
wobei die Summe der Gewichtsprozente aus den Komponenten der Estermischungen 100 % ergibt und
worin
- R¹: ein geradkettiger oder verzweigter C₁- bis C₂₀-Alkylrest,
- R²: ein optional mit C₁- bis C₄-Alkyl oder Halogen substituierter Benzylrest,
- Z: ein m-wertiger gesättigter oder ungesättigter, geradkettiger, verzweigter, cyclischer oder polycyclischer C₂- bis C ₁₀- Kohlenwasserstoffrest,
- m: eine Zahl von 2 bis 4 und
- n: eine ganze Zahl von 1 bis m bedeuten.

Bevorzugt steht der Rest R¹ für n-Butyl, 2-Ethylhexyl oder Isononyl.

Bevorzugt steht der Rest R² für Benzyl (= -CH₂-Ph).

Bevorzugt leitet sich der Rest Z strukturell ab von der Phthal-, Terephthal-, Pyromellit-, Trimellit-, Malein-, Fumar-, Adipin-, Azelain- oder Sebazinsäure.

In einer besonders bevorzugten Form der Erfindung handelt es sich bei dem Ester der Struktur Z-(COOR²)ₘ₋ₙ(COOR¹)ₙ mit m = n = 2 um Diisononylphthalat und für m = 2 mit n = 1 um Isononylbenzylphthalat und bei dem Ester der Struktur Z-(COOR²)ₘ um Dibenzylphthalat.

In einer alternativen, besonders bevorzugten Form der Erfindung handelt es sich bei dem Ester der Struktur Z-(COOR²)ₘ₋ₙ(COOR¹)ₙmit m = n = 3 um Tributyltrimellitat und für m = 3 mit n = 1 bzw. 2 um Benzyldibutyltrimellitat bzw. Dibenzyl butyltrimellitat und bei dem Ester der Struktur Z-(COOR²)ₘ um Tribenzyltrimellitat.

Die erfindungsgemäßen Estermischungen lassen sich durch Mischen der an sich bekannten Komponenten im angegebenen Verhältnis herstellen. Enthalten die erfindungsgemäßen Estermischungen mehr als zwei Komponenten, so können sie vorteilhaft auch durch eine Mischveresterung hergestellt werden. Die Erfindung betrifft daher auch ein Verfahren zur Herstellung von Estermischungen mit
a) 2 - 98 Gew.-% mehrerer Ester der Struktur Z-(COOR²)ₘ₋ₙ(COOR¹)ₙ und
b) 2 - 98 Gew.-% eines Esters der Struktur Z-(COOR²)ₘ
wobei die Summe der Gewichtsprozente aus den Komponenten der Estermischungen 100 % ergibt und
worin
- R¹: ein geradkettiger oder verzweigter C₁- bis C₂₀-Alkylrest,
- R²: ein optional mit C₁- bis C₄-Alkyl oder Halogen substituierter Benzylrest,
- Z: ein m-wertiger gesättigter oder ungesättigter, geradkettiger, verzweigter, cyclischer oder polycyclischer C₂- bis C ₁₀- Kohlenwasserstoffrest,
- m: eine Zahl von 2 bis 4 und
- n: eine ganze Zahl von 1 bis m bedeuten,
dadurch gekennzeichnet, dass in einem Verfahrensschritt mindestens eine aromatische oder aliphatische Polycarbonsäure oder ein Derivat davon, mindestens ein Benzylalkohol und mindestens ein aliphatischer Alkohol bei Temperaturen von 50 bis 300 °C und Drücken von 2 mbar bis 4 bar optional in Gegenwart eines Katalysators miteinander umgesetzt werden und dabei das Reaktionswasser durch geeignete Maßnahmen aus der Mischung entfernt wird.

Mehrere Ester bedeuten in der vorliegenden Anmeldung > 1 bis m, bevorzugt 2 bis 4. Die aromatische oder aliphatische Polycarbonsäure kann als solche oder in Form eines Derivats, wie zum Beispiel in Form eines Anhydrids, Esters, Säurechlorids in das erfindungsgemäße Verfahren eingesetzt werden. In einer bevorzugten Ausführungsform der Erfindung werden die Polycarbonsäuren und/oder ihre Anhydride eingesetzt.

Die Umsetzung kann durch Entfernen des Reaktionswassers vervollständigt werden. Dies kann beispielsweise mittels Schleppmittel wie Xylol, Toluol oder vorzugsweise auch durch Verwendung eines Alkohols, der selbst an der Reaktion teilnimmt, erreicht werden. Besonders bevorzugt erfolgt die Wasserentfernung im Bereich von Temperaturen im Bereich von 50 bis 250 °C und Drücken von 200 mbar bis 4 bar.

Die bei dem erfindungsgemäßen Verfahren verwendeten Reaktionspartner sind (1) aromatische oder aliphatische Polycarbonsäuren oder deren Anhydride, (2) Benzylalkohole, (3) Monohydroxyaliphatische Alkohole und optional (4) Katalysatoren. Nachfolgend erfolgt eine detaillierte Beschreibung dieser Reaktionspartner.

Erfindungsgemäß einzusetzende Anhydride können entweder die der aliphatischen oder der aromatischen Säuren sein und können gesättigt oder ungesättigt sein. Beispiele geeigneter Ahydride sind die Anhydride von Phthal-, Pyromellith-, Trimellith-, Malein-, Bernstein- und Glutarsäure. Für die Zwecke des erfindungsgemässen Verfahrens kann das Anhydrid als Schmelze, als Feststoff, wie z. B. in der Form von Flocken, oder als Lösung zu einer Reaktionszone zugegeben werden.

Erfindungsgemäß einzusetzende Benzylalkohole sind beispielsweise Benzylalkohol, ebenso wie Alkyl-substituierte Benzylalkohole, zum Beispiel Methylbenzylalkohol, Ethylbenzylalkohol, Dimethylbenzylalkohol und ähnliche, und die Halogen-substituierten Benzylalkohole, zum Beispiel Chlorbenzylalkohol, Dichlorbenzylalkohol, Trichlorbenzylalkohol, Brombenzylalkohol, Dibrombenzylalkohol, und ähnliche.

Erfindungsgemäß einzusetzende Alkohole sind einwertige aliphatische Alkohole, wie Methylalkohol, Ethylalkohol, Propylalkohol, Isopropylalkohol, Butylalkohole, zum Beispiel n-Butylalkohol und sek. Butylalkohol, Isobutylalkohol, Amylalkohol, Hexylalkohole, zum Beispiel n-Hexylalkohol, 1,4-Dimethylbutylalkohol, n-Heptylalkohol, Octylalkohole, zum Beispiel Isooctylalkohole, n-Octylalkohol, 2-Ethylhexanol, n-Nonylalkohol, Isononylalkohole, Decylalkohole, zum Beispiel n-Decylalkohol, Isodecylalkohol, 2-Propylheptanol, Dodecylalkohol, Tridecylalkohol, Tetradecylalkohol, Pentadecylalkohol, Cetylalkohol, Octadecylalkohol, und Eicosylalkohol; cycloaliphatische Alkohole, wie Cyclopropylcarbinol, Cyclobutylalkohol, Cyclopentylalkohol, Methylcyclopentylalkohol, Dimethylcyclopentylalkohol, Äthylcyclopentylalkohol, Cyclohexylalkohol, Methylcyclohexylalkohol, Dimethylcyclohexylalkohol und Cyclooctylalkohol; und ungesättigte aliphatische Alkohole, wie Allylalkohol, Crotylalkohol und ähnliche. Darüber hinaus sind die gesamten verschiedenen isomeren Formen dieser Alkohole und Gemische derselben zur Verwendung in dem erfindungsgemäßen Verfahren geeignet. Darüber hinaus berührt die Herkunft des Alkohols nicht das Verfahren, und so können beispielsweise aliphatische Alkohole, welche von Ein- oder Zwei-Stufen-Oxoverfahren, aus der Hydratisierung von Olefinen oder aus der katalytischen Dehydrierung von Kokosnussöl herrühren, verwendet werden und sind tatsächlich wegen ihrer Erreichbarkeit wünschenswert.

Gegebenenfalls erfindungsgemäß einzusetzende Katalysatoren sind prinzipiell alle Veresterungskatalysatoren. Besonders bevorzugt werden Katalysatoren der Formel

MXₙ

mit
- M: ausgewählt aus der Gruppe Titan, Zirkonium, Vanadium, Aluminium, Eisen, Zinn,
- X: ausgewählt aus der Gruppe, CO₃²⁻, Cl, Br, I; OR mit R ausgewählt aus Methyl, Ethyl, n-Propyl, i-Propyl, n-Butyl, i-Butyl, t-Butyl; Carboxylate insbesondere Hexanoat, Heptanoat, Octanoat, 2-Ethylhexanoat, Stearat, Palmitat, Oxalat
- n: Oxidationszahl des Metalls, bevorzugt 2, 3 oder 4,
eingesetzt.

Alternativ können auch starke Brönstedsäuren wie Schwefelsäure, saure Sulfate, wie z.B. Methylsulfat, Ethylsulfat, Propylsulfat, Butylsulfat, Hexylsulfat aber auch KHSO₄ bzw. NaHSO₄, aromatische Sulfonsäuren insbesondere para-Toluolsulfonsäure, Benzolsulfonsäure erfolgreich eingesetzt werden.

Die Erfindung umfasst auch die Verwendung der Estermischungen als Weichermacher für Kunststoffe wie Polyvinylchlorid, Vinylchlorid-basierte Copolymere, Polyvinylidenchlorid, Polyvinylacetale, Polyacrylate, Polyamide, Polyurethane, Polylactide, Polymilchsäuren, Cellulose und seine Derivate, Kautschukpolymere wie Acrylnitril-Butadien-Kautschuk, hydrierter Acrylnitril-Butadien-Kautschuk, Chloroprenkautschuk, chloriertes Polyethylen, Chlorsulfonylpolyethylen, Ethylen-Propylen-Kautschuk, Acrylatkautschuk und/oder Epichlorhydrinkautschuk. Bevorzugt ist Polyvinylchlorid.

Das Polyvinylchlorid wird dabei vorzugsweise durch Homopolymerisation aus Vinylchlorid nach dem Fachmann bekannten Methoden wie der Suspensions-, der Emulsions- oder der Massepolymerisation hergestellt. Die erfindungsgemäßen Estermischungen werden vorzugsweise in Gemischen mit 20 bis 99 % Polyvinylchlorid, bevorzugt 45 bis 95 % Polyvinylchlorid, besonders bevorzugt 50 bis 90 % Polyvinylchlorid eingesetzt. Diese Gemische werden Weich-Polyvinylchlorid genannt und können neben den erfindungsbemäßen Estermischungen und Polyvinylchlorid auch noch andere geeignete Zusatzstoffe enthalten. Beispielsweise können Stabilisatoren, Gleitmittel, Füllstoffe, Pigmente, Flammschutzmittel, Lichtstabilisatoren, Treibmittel, polymere Verarbeitungeshilfsmittel, Schlagzähverbesserer, optische Aufheller, Antistatika oder Biostabilisatoren enthalten sein.

Die Kunststoffe erhalten vorzugsweise auch Zusatzstoffe wie Stabilisatoren, Gleitmittel, Füllstoffe, Pigmente, Flammschutzmittel, Lichtstabilisatoren, Treibmittel, polymere Verarbeitungeshilfsmittel, Schlagzähverbesserer, optische Aufheller, Antistatika und/oder Biostabilisatoren sowie Mischungen davon.

Im Folgenden werden einige geeignete Zusatzstoffe näher beschrieben. Die aufgeführten Beispiele stellen jedoch keine Einschränkung der erfindungsgemässen Gemische dar, sondern dienen lediglich der Erläuterung. Alle Angaben zum Gehalt sind Gewichts-%-Angaben.

Stabilisatoren neutralisieren die während und/oder nach der Verarbeitung des Polyvinylchlorid abgespaltene Salzsäure. Als Stabilisatoren kommen alle üblichen Polyvinylchlorid-Stabilisatoren in fester und flüssiger Form in Betracht, beispielsweise übliche Epoxy/Zink-, Ca/Zn-, Ba/Zn-, Pb- oder Sn-Stabilisatoren sowie auch säurebindende Schichtsilikate wie Hydrotalcit. Die erfindungsgemässen Estermischungen können in Gemischen mit einem Gehalt an Stabilisatoren von 0,05 bis 7 %, bevorzugt 0,1 bis 5 %, besonders bevorzugt von 0,2 bis 4 % und insbesondere von 0,5 bis 3 % eingesetzt werden.

Gleitmittel sollen zwischen den Polyvinylchlorid-Partikeln wirksam werden und Reibungskräften beim Mischen, Plastifizieren und Verformen entgegenwirken. Als Gleitmittel können die erfindungsgemässen Gemische alle für die Verarbeitung von Kunststoffen üblichen Gleitmittel enthalten. Beispielsweise kommen in Betracht Kohlenwasserstoffe, wie Öle, Paraffine und PE-Wachse, Fettalkohole mit 6 bis 20C- Atomen, Ketone, Carbonsäuren, wie Fettsäuren und Montansäuren, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettige Carbonsäuren als Säurekomponente. Die erfindungsgemässen Estermischungen können in Gemischen mit einem Gehalt an Gleitmitteln von 0,01 bis 10 %, bevorzugt 0,05 bis 5%, besonders bevorzugt von 0,1 bis 3 % und insbesondere von 0,2 bis 2 % eingesetzt werden.

Füllstoffe beeinflussen vor allem die Druck-, Zug- und Biegefestigkeit sowie die Härte und Wärmeformbeständigkeit von weichgemachtem Polyvinylchlorid oder Polyvinylbromid in positiver Weise. Im Rahmen der Erfindung können die Gemische auch Füllstoffe wie beispielsweise Russ und andere anorganische Füllstoffe, wie natürliche Calciumcarbonate, beispielsweise Kreide, Kalkstein und Marmor, synthetische Calciumcarbonate, Dolomit, Silikate, Kieselsäure, Sand, Diatomeenerde, Aluminiumsilikate, wie Kaolin, Glimmer und Feldspat. Vorzugsweise werden als Füllstoffe Calciumcarbonate, Kreide, Dolomit, Kaolin, Silikate, Talkum oder Ruß eingesetzt. Die erfindungsgemässen Estermischungen können in Gemischen mit einem Gehalt an Füllstoffen von 0,01 bis 80 %, bevorzugt 0,1 bis 60 %, besonders bevorzugt von 0,5 bis 50 % und insbesondere von 1 bis 40 % eingesetzt werden.

Die mit den erfindungsgemässen Estermischungen zubereiteten Gemische können auch Pigmente enthalten, um das erhaltene Produkt an unterschiedliche Einsatzmöglichkeiten anzupassen. Im Rahmen der vorliegenden Erfindung können sowohl anorganische Pigmente als auch organische Pigmente eingesetzt werden. Als anorganische Pigmente können beispielsweise Cadmium-Pigmente, wie CdS, Kobalt-Pigmente, wie CoO/Al₂O₃, und Chrom-Pigmente, beispielsweise Cr₂0₃, verwendet werden. Als organische Pigmente kommen beispielsweise Monoazopigmente, kondensierte Azopigmente, Azomethinpigmente, Anthrachinonpigmente, Chinacridone, Phthalocyaninpigmente, Dioxa-zinpigmente und Anilinpigmente in Betracht. Die erfindungsgemässen Estermischungen können in Gemischen mit einem Gehalt an Pigmenten von 0,01 bis 10 %, bevorzugt 0,05 bis 5 %, besonders bevorzugt von 0,1 bis 3 % und insbesondere von 0,5 bis 2 % eingesetzt werden.

Um die Entflammbarkeit zu vermindern und die Rauchentwicklung beim Verbrennen zu verringern, können die erfindungsgemässen Gemische auch Flammschutzmittel enthalten. Als Flammschutzmittel können beispielsweise Antimontrioxid, Phosphatester, Chlorparaffin, Aluminiumhydroxid, Borverbindungen, Molybdäntrioxid, Ferrocen, Calciumcarbonat oder Magnesiumcarbonat verwendet werden. Die erfindungsgemässen Estermischungen können in Gemischen mit einem Gehalt an Flammschutzmittel von 0,01 bis 30 %, bevorzugt 0,1 bis 25 %, besonders bevorzugt von 0,2 bis 20 % und insbesondere von 0,5 bis 15 % eingesetzt werden.

Um Artikel, die aus einem die erfindungsgemäßen Estermischungen enthaltenden Gemisch hergestellt wurden, vor einer Schädigung im Oberflächenbereich durch den Einfluss von Licht zu schützen, können die Gemische auch Lichtstabilisatoren enthalten. Es können im Rahmen der vorliegenden Erfindung beispielsweise Hydroxybenzophenone oder Hydroxyphenylbenzotriazole eingesetzt werden. Die erfindungsgemäßen Estermischungen können in Gemischen mit einem Gehalt an Lichtstabilisatoren von 0,01 bis 7 %, bevorzugt 0,1 bis 5 %, besonders bevorzugt von 0,2 bis 4 % und insbesondere von 0,5 bis 3 % eingesetzt werden. Alle % Angaben in dieser Anmeldung sind Gew.-% sofern nichts anderes angegeben ist.

Die Kunststoffe enthalten vorzugsweise auch weitere Weichmacher wie Monoalkylester der Benzoesäure, Benzoesäurediester von Mono-, Di-, Tri- oder Polyalkylenglycolen, Dialkylester aliphatischen Disäuren, Dialkylester aromatischer Disäuren, Trialkylester aromatischer Trisäuren, Phenylester von Alkansulfonsäuren, Alkyl- oder Arylester der Phosphorsäure, Polyester aus Dicarbonsäuren sowie Mischungen davon.

Beispiele für weitere Weichmacher sind
- die Monoalkylester der Benzoesäure, wie zum Beispiel Isononylbenzoat,
- die Benzoesäurediester von Mono-, Di-, Tri- oder Polyalkylenglycolen, wie zum Beispiel Propylenglycoldibenzoat, Diethylenglycoldibenzoat, Dipropylenglycoldibenzoat, Triethylenglycoldibenzoat oder Polyethylenglycoldibenzoat und insbesondere Mischungen davon,
- die Dialkylester aliphatischer Disäuren, wie zum Beispiel Di-(2-ethylhexyl)-adipat, Diisononyladipat, Di-(2-ethylhexyl)-sebazat, Di-(2-ethylhexyl)-azelat, Diisononylcyclohexan-1,2-dicarboxylat,
- die Dialkylester aromatischer Disäuren, wie zum Beispiel Di-(2-ethylhexyl)-phthalat, Diisononylphthalat, Diisodecylphthalat, Benzylbutylphthalat, Benzylisooctylphthalat, Benzylisononylphthalat,
- die Trialkylester aromatischer Trisäuren, wie zum Beispiel Trioctyltrimellitat,
- die Phenylester von Alkansulfonsäuren, wie zum Beispiel das Produkt Mesamoll^{®} der LANXESS Deutschland GmbH,
- die Alkyl- oder Arylester der Phosphorsäure, wie zum Beispiel Tri-(2-ethylhexyl)-phosphat, Diphenyl-2-ethylhexyl-phosphat, Diphenylkresylphosphat oder Trikresylphosphat,
- Polyester, die zum Beispiel aus Dicarbonsäuren wie Adipinsäure oder Phthalsäure und Diolen wie 1,2-Propandiol, 1,3-Butandiol, 1,4-Butandiol oder 1,6-Hexandiol hergestellt werden können.

Im Rahmen der Erfindung können die erfindungsgemäßen Estermischungen auch in Gemischen eingesetzt werden, die weitere Kunststoffe ausgewählt aus der Gruppe bestehend aus Homo- und Copolymeren auf Basis von Ethylen, Propylen, Butadien, Vinylacetat, Glycidylacrylat, Glycidylmethacrylat, Acrylaten und Methacrylaten mit Alkoholkomponenten von verzweigten oder unverzweigten C₁- bis C₁₀-Alkoholen, Styrol oder Acrylnitril enthalten. Zu nennen sind beispielsweise Polyacrylate mit gleichen oder verschiedenen Alkoholresten aus der Gruppe der C₄- bis C₈-Alkohole, besonders des Butanols, Hexanols, Octanols und 2-Ethylhexanols, Polymethylmethacrylat, Methylmethacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Rubber, Styrol-Butadien-Elastomere und Methylmethacrylat-Styrol-Butadien-Copolymere.

Die mit den erfindungsgemäßen Estermischungen hergestellten Kunststoffgemische sind beispielsweise nützlich zur Herstellung von Rohrleitungen, Kabeln, Draht-Ummantelungen, im Innenausbau, im Fahrzeug- und Möbelbau, in Bodenbelägen, medizinischen Artikeln, Lebensmittelverpackungen, Dichtungen, Folien, Verbundfolien, Folien für Verbundsicherheitsglas, insbesondere für den Fahrzeug-Bereich und den Architektur-Sektor, Kunstleder, Spielzeug, Verpackungsbehältern, Klebebandfolien, Bekleidung, Beschichtungen, sowie Fasern für Gewebe.

Die erfindungsgemäßen Estermischungen sind wegen ihrer sehr niedrigen Lösetemperaturen gut verarbeitbar und weisen eine geringe Flüchtigkeit auf.

Die Weichmacher und das Verfahren dieser Erfindung werden durch die nachfolgenden, den Erfindungsbereich nicht einschränkenden Beispiele erläutert. [Angaben in Flächenprozenten].

### Bestimmung der Lösetemperatur

Zur Bestimmung der Lösetemperatur wurden 48 g des zu prüfenden Weichmachers im Becherglas mit 2 g Polyvinylchlorid, Typ Vinnol^{®} H70, Korngröße < 315 µm, und 2 Tropfen Irgastab^{®} 17 M verrührt. Die Suspension wurde unter Rühren mit 2 - 3 °C pro Minute solange aufgeheizt, bis eine Temperatur erreicht ist, bei der 3 Minuten hintereinander keinerlei Anstieg in der Anzeige einer hinter dem Becherglas positionierten Photozelle mehr zu verzeichnen ist und das Polyvinylchlorid gelöst ist.

### Bestimmmung der Flüchtigkeit

Als Maß für die Flüchtigkeit wurde die Menge der kondensierbaren Bestandteile wie folgt bestimmt. Dabei werden 10 g des Weichmachers in ein thermostatiertes, zylindrisches Gefäß gegeben (Fogging Test Gerät N8-FPG). Als Kondensationsflächen dient eine gekühlte Aluminiumfolie, deren Gewicht zuvor bestimmt wurde. Anschließend wird der versiegelte Zylinder für 16 h auf 100 °C erwärmt. Dann wird die Aluminiumfolie entnommen. Nach Lagerung im Exsikkator für 4 h wird durch Differenzwägung die Gewichtszunahme ermittelt. Die Flüchtigkeit wird durch Doppelbestimmung ermittelt.

### Beispiele 1 bis 10: Herstellung von Estermischungen

In einem mit Stickstoffgas inertisierten 2 1 Dreihalsrundkolben mit Rückflusskühler, Innenthermometer und Rührer wurden Benzylalkohol und Isononanol (Verhältnis Benzylalkohol/Isononanol siehe Tabelle 1; 2,05 Moläquivalente Gesamtalkohol bez. Phthalsäureanhydrid) vorgelegt und anschließend geschmolzenes Phthalsäureanhydrid zugetropft, wobei die Reaktionstemperatur bei 95 °C lag. Anschließend wurde für weitere 16 h bei dieser Temperatur gehalten. Nach Abkühlung wurden Xylol (15 Gew.-% bezogen auf den Gesamtansatz) zugegeben und ein Wasserabscheider eingebaut. Der Reaktor wurde auf 100 °C erwärmt und bei Erreichen der Temperatur Titantetra-n-butylat (0,25 Gew.-% bezogen auf das Gesamtansatz) zugegeben. Der Reaktionsverlauf wird mittels Titration [Säurezahl (SZ: mg KOH/gSubstanz); Korrektur Xylolverdünnung] und durch die Messung der abgeschiedenen Wassermenge verfolgt. Die Reaktionstemperatur wurde im Verlauf von 15-20 h von 140 °C auf 180 °C erhöht.

Nach Erreichen einer SZ (= Säurezahl) von <1 wurde der Versuch beendet und das Produkt mit Wasserdampf bei 100 °C behandelt. Die Wasserdampfdestillation wurde beendet, nachdem das doppelte Volumen des organischen Anteils kondensierte. Anschließend wurden bei 140 °C und einem Druck von 4 mbar alle flüchtigen Bestandteile entfernt und der Rückstand nach Versetzten mit einer Mischung aus MgO/Aktivkohle filtriert. Die Produkte wurden mittels HPLC auf ihre Zusammensetzung analysiert und anwendungstechnisch als Weichmacher in Polyvinylchlorid geprüft.

**Tabelle 1**

| **Beispiel** | **1** | **2** | **3** | **4** | **5** | **6** | **7** | **8** | **9** | **10** |
|---|---|---|---|---|---|---|---|---|---|---|
| Nonanol/Benzylalkohol [mol/mol] | 0,98 | 0,51 | 1,00 | 1,03 | 1,00 | 1,00 | 1,00 | 1,22 | 1,62 | 1,00 |

| **Zusammensetzung [%]** | | | | | | | | | | |
|---|---|---|---|---|---|---|---|---|---|---|
| Dibenzylphthalat | 26,00 | 37,76 | 30,44 | 30,57 | 30,68 | 28,04 | 30,06 | 23,86 | 17,16 | 28,3 |
| Isononylbenzylphthalat | 48,82 | 45,87 | 49,87 | 49,64 | 50,96 | 52,26 | 51,47 | 50,08 | 48,13 | 49,2 |
| Diisononylphthalat | 25,18 | 16,37 | 19,69 | 19,79 | 18,36 | 19,71 | 18,47 | 26,06 | 34,72 | 22,5 |

### Beispiel 11: Herstellung einer Estermischung

In einem mit Stickstoffgas inertisierten 2 1 Dreihalsrundkolben mit Rückflusskühler, Innenthermometer und Rührer wurden Benzylalkohol und Butanol (Verhältnis Benzylalkohol: n-Butanol = 1:1 Mol/Mol; 3,9 Moläquivalente Gesamtalkohol bez. Trimellitsäureanhydrid) vorgelegt und anschließend Trimellitsäureanhydrid portionsweise zugegeben, wobei die Reaktionstemperatur bei 95 °C lag. Anschließend wurde die Lösung für 1 h bei 125 °C gehalten. Nach Abkühlung wurden Xylol (15 Gew.-% bezogen auf den Gesamtansatz) zugegeben und ein Wasserabscheider eingebaut. Der Reaktor wurde auf 100 °C erwärmt und bei Erreichen der Temperatur Titantetra-n-butylat (0,28 Mol-% bezogen auf Trimellitsäureanhydrid) zugegeben. Der Reaktionsverlauf wird mittels Titration [Säurezahl (SZ: mg KOH/gSubstanz); Korrektur Xylolverdünnung] und durch die Messung der abgeschiedenen WasserButanol-Menge verfolgt. Die Reaktionstemperatur wurde innerhalb von 23 h auf 165 °C erhöht.

Nach Erreichen einer SZ von 2,5 wurde der Versuch beendet und das Produkt mit Wasserdampf bei 100 °C behandelt. Die Wasserdampfdestillation wurde beendet, nachdem das doppelte Volumen des organischen Anteils kondensierte. Anschließend wurden bei 140 °C und einem Druck von 20 mbar alle flüchtigen Bestandteile entfernt und der Rückstand nach Versetzen mit einer Mischung aus Na₂CO₃/Aktivkohle filtriert. Die Produkte wurden mittels GCMS auf ihre Zusammensetzung analysiert und anwendungstechnisch als Weichmacher in Polyvinylchlorid geprüft.

**Tabelle 2**

| **Beispiel** | **11** |
|---|---|
| ButanolBenzylalkohol [mol/mol] | 1 |
| Ausbeute [%] | 89,5 |

| **Zusammensetzung %** | |
|---|---|
| Tributyltrimellitat | 7,7 |
| Dibutylbenzyltrimellitat | 30,6 |
| Monobutyldibenzyltrimellitat | 39,2 |
| Tribenzyltrimellitat | 11,5 |

### Beispiele 12 und 13: Herstellung einer Estermischung

In einem mit Stickstoffgas inertisierten 2 1 Dreihalsrundkolben mit Rückflusskühler, Innenthermometer und Rührer wurden Benzylalkohol und i-Nonanol (Verhältnis Benzylalkohol: i-Nonanol s. Tabelle 3; 3,05 Moläquivalente Gesamtalkohol bez. Trimellitsäureanhydrid) vorgelegt und anschließend Trimellitsäureanhydrid (1.0 Moläquivalent) portionsweise zugegeben, wobei die Reaktionstemperatur bei 95 °C lag. Anschließend wurde die Lösung für 2 h bei 125 °C gehalten. Nach Abkühlung wurden Xylol (15 Gew.-% bezogen auf das Gesamtansatz) zugegeben und ein Wasserabscheider eingebaut. Der Reaktor wurde auf 100 °C erwärmt und bei Erreichen der Temperatur Titantetra-n-butylat (0,28 Mol-% bezogen auf Trimellitsäureanhydrid) zugegeben. Der Reaktionsverlauf wird mittels Titration [Säurezahl (SZ: mgKOH/gSubstanz); Korrektur Xylolverdünnung] und durch die Messung der abgeschiedenen Wassermenge verfolgt. Die Reaktionstemperatur wurde im Verlauf von 3 h von 120 °C auf 170 °C erhöht.

Nach Erreichen einer SZ von kleiner 2 wurde der Versuch beendet und das Produkt mit Wasserdampf bei 100 °C behandelt. Die Wasserdampfdestillation wurde beendet, n achdem das doppelte Volumen des organischen Anteils kondensierte. Anschließend wurden bei 140 °C und einem Druck von 10 mbar alle flüchtigen Bestandteile entfernt und der Rückstand nach Versetzten mit einer Mischung aus MgO/Aktivkohle filtriert. Die Produkte wurden mittels GCMS auf ihre Zusammensetzung analysiert und anwendungstechnisch als Weichmacher in Polyvinylchlorid geprüft.

**Tabelle 3**

| **Beispiel** | **12** | **13** |
|---|---|---|
| Nonanol/Benzylalkohol [mol/mol] | 0,5 | 2 |
| Ausbeute [%]: | 90 | 91 |

| **Zusammensetzung [%]** | | |
|---|---|---|
| Tri-isononylyltrimellitat | 2,5 | 20,3 |
| Di-isononylbenzyltrimellitat | 26,2 | 51,0 |
| Mono-isononyldibenzyltrimellitat | 44,0 | 24,1 |
| Tribenzyltrimellitat | 27,2 | 4,5 |

### Beispiele 14 bis 17

Nach der für die Beispiele 12 und 13 angegebenen Herstellvorschrift wurden unter Einsatz der in Tabelle 4 angegebenen Ausgangsstoffe und Molverhältnisse die Estermischungen der Beispiele 14 bis 17 hergestellt.

**Tabelle 4**

| **Beispiel** | **14** | **15** | **16** | **17** |
|---|---|---|---|---|
| Alkohol R¹-OH | 2-Ethylhexanol | Isononanol | 2-Ethylhexanol | Isodecanol |
| Benzylalkohol R²-OH | Ph-CH₂-OH | Ph-CH₂-OH | Ph-CH₂-OH | Ph-CH₂-OH |
| R¹-OH/R²-OH mol/mol | 1,0 | 1,0 | 1,0 | 1,0 |
| Säure Z(COOH)ₘ | Pyromellitsäure | Adipinsäure | Azelainsäure | Sebazinsäure |
| m | 4 | 2 | 2 | 2 |
| Gesamtalkohol/Säure mol/mol | 4,05 | 2,1 | 2,05 | 2,1 |

| **Komponenten der Estermischung** | | | | |
|---|---|---|---|---|
| Z-(COOR²)ₘ | Tetrabenzylpyromellitat | Dibenzyladipat | Dibenzylazelat | Dibenzylsebazat |
| Z-(COOR²)ₘ₋ₙ(COOR¹)ₙ mit n = 1 | Tribenzyl-2-ethylhexyl-pyromellitat | Benzylisononyl-adipat | Benzyl-2-ethylhexylazelat | Benzylisodecylsebazat |
| Z-(COOR²)ₘ₋ₙ(COOR¹)ₙ mit n = 2 | Dibenzyl-di-2-ethylhexyl-pyromellitat | Diisononyladipat | Di-2-ethylhexylazelat | Diisodecylsebazat |
| Z-(COOR²)ₘ₋ₙ(COOR¹)ₙ mit n = 3 | Benzyl-tri-2-ethylhexyl-pyromellitat | | - | - |
| Z-(COOR²)ₘ₋ₙ(COOR¹)ₙ mit n = 4 | Tetra-2-ethylhexyl-pyromellitat | - | - | - |

**Stoffeigenschaften der Estermischungen aus den Beispielen 10 bis 13**

**Tabelle 5**

| **Beispiel** | **Lösetemperatur (°C)** | **Viskosität (mPas)** | **Pour-Point (°C)** | **Kondensat (mg)** |
|---|---|---|---|---|
| **10** | 123 | 78 | -32 | 31 |
| **11** | 120 | Feststoff | Feststoff | 1,1 |
| **12** | 142 | 381 | -26 | 1,1 |
| **13** | 142 | 749 | 1 | 0,4 |
| | | | | |

| **Nicht erfindungsgemäß:** | | | | |
|---|---|---|---|---|
| Dibenzylphthalat | 145 | 221 | -12 | > 30 Rückfluß |
| Santicizer^{®} 261 A | 119 | 85 | -35 | 1,40 |
| Unimoll^{®} BB | 110 | 62 | -40 | 36 |
| Vestinol^{®} 9 | 132 | 82 | -54 | 1,46 |
| Uraplast^{®} 525 | 160 | 114 | -43 | 0,13 |

| | | | | |
|---|---|---|---|---|
| Santicizer^{®} 261 A: Benzylisononylphthalat; Unimoll^{®} BB: Benzylbutylphthalat; Vestinol^{®} 9 : Diisononylphthalat; Uraplast^{®} 525: C₈-/C₁₀-Trialkyltrimellitat von linearen C₈-/C₁₀-Alkoholen. | | | | |

Aus den Beispielen ist leicht zu ersehen, dass das erfindungsgemäße Verfahren in hohen Ausbeuten unter Vermeidung von Benzylchlorid und ohne salzhaltige Abwässer erlaubt, die erfindungsgemäßen Weichmacher herzustellen. Die Lösetemperaturen der erfindungsgemäßen Weichmacher sind niedriger als die Lösetemperaturen der in ihnen enthaltenen reinen Polyalkylester oder Polybenzylester. Dabei ist ihre Flüchtigkeit in einer Größenordnung, wie sie für vergleichbare, kommerziell eingesetzte Weichmacher gefunden wird.

## Patentansprüche

1. Estermischung enthaltend
a) 2-98 Gew.-% eines oder mehrerer Ester der Struktur Z-(COOR²)ₘ₋ₙ(COOR¹)ₙ und
b) 2 - 98 Gew.-% eines Esters der Struktur Z-(COOR²)ₘ
wobei die Summe der Gewichtsprozente aus den Komponenten der Estermischungen 100 % ergibt und
worin
R¹ ein geradkettiger oder verzweigter C₁- bis C₂₀-Alkylrest,
R² ein optional mit C₁- bis C₄-Alkyl oder Halogen substituierter Benzylrest,
Z ein m-wertiger gesättigter oder ungesättigter, geradkettiger, verzweigter, cyclischer oder polycyclischer C₂- bis C ₁₀- Kohlenwasserstoffrest,
m eine Zahl von 2 bis 4 und
n eine ganze Zahl von 1 bis m bedeuten.

2. Estermischung gemäß Anspruch 1, **dadurch gekennzeichnet, dass** R² für Benzyl steht.

3. Estermischung gemäß Anspruch 1 oder 2, **dadurch gekennzeichnet, dass** R¹ für n-Butyl, 2-Ethylhexyl oder Isononyl steht.

4. Estermischung nach Anspruch 1, **dadurch gekennzeichnet, dass** es sich bei dem Ester der Struktur Z-(COOR²)ₘ₋ₙ(COOR¹)ₙ mit m = n = 2 um Diisononylphthalat und für m = 2 mit n = 1 um Isononylbenzylphthalat und bei dem Ester der Struktur Z-(COOR²)ₘ um Dibenzylphthalat handelt.

5. Estermischung nach Anspruch 1, **dadurch gekennzeichnet, dass** es sich bei dem Ester der Struktur Z-(COOR²)ₘ₋ₙ(COOR¹)ₙ mit m = n = 3 um Tributyltrimellitat und für m = 3 mit n = 1 bzw. 2 um Benzyldibutyltrimellitat bzw. Dibenzylbutyltrimellitat und bei dem Ester der Struktur Z-(COOR²)ₘ um Tribenzyltrimellitat handelt.

6. Verfahren zur Herstellung von Estermischungen enthaltend
a) 2-98 Gew.-% mehrerer Ester der Struktur Z-(COOR²)ₘ₋ₙ(COOR¹)ₙ und
b) 2-98 Gew.-% eines Esters der Struktur Z-(COOR²)ₘ
wobei die Summe der Gew.-% aus den Komponenten der Estermischungen 100 % ergibt und
worin
R¹ ein geradkettiger oder verzweigter C₁- bis C₂₀-Alkylrest,
R² ein optional mit C₁- bis C₄-Alkyl oder Halogen substituierter Benzylrest,
Z ein m-wertiger gesättigter oder ungesättigter, geradkettiger, verzweigter, cyclischer oder polycyclischer C₂- bis C ₁₀- Kohlenwasserstoffrest,
m eine Zahl von 2 bis 4 und
n eine ganze Zahl von 1 bis m
bedeuten, **dadurch gekennzeichnet, dass** in einem Verfahrensschritt mindestens eine aromatische oder aliphatische Polycarbonsäure oder ein Derivat davon, mindestens ein Benzylalkohol und mindestens ein aliphatischer Alkohol bei Temperaturen von 50 bis 300 °C und Drücken von 2 mbar bis 4 bar optional in Gegenwart eines Katalysators miteinander umgesetzt werden und dabei das Reaktionwasser durch geeignete Maßnahmen aus der Mischung entfernt wird.

7. Verfahren gemäß Anspruch 6, **dadurch gekennzeichnet, dass** Polycarbonsäuren oder deren Derivate, ausgewählt aus der Gruppe bestehend aus Phthalsäure, Terephthalsäure, Pyromellithsäure, Trimellitsäure, Sebazinsäure, Azelainsäure, Adipinsäure, Maleinsäure und Fumarsäure eingesetzt werden.

8. Verfahren gemäß der Ansprüche 6 oder 7, **dadurch gekennzeichnet, dass** ein substituierter oder unsubstituierter Benzylalkohol R²-OH im Gemisch mit einem oder mehreren aliphatischen Alkoholen, R¹-OH, worin R¹ ausgewählt ist aus der Gruppe bestehend aus verzweigten und unverzweigten Alkyl-, Cycloalkyl- und Oxalkylenresten, eingesetzt wird.

9. Verwendung der Estermischungen gemäß den Ansprüchen 1 bis 5 und der nach den Verfahren gemäß Ansprüchen 6 bis 8 hergestellten Estermischungen als Weichermacher für Kunststoffe wie Polyvinylchlorid, Vinylchlorid-basierte Copolymere, Polyvinylidenchlorid, Polyvinylacetale, Polyacrylate, Polyamide, Polyurethane, Polylactide, Polymilchsäuren, Cellulose und seine Derivate, Kautschukpolymere wie Acrylnitril-Butadien-Kautschuk, hydrierter Acrylnitril-Butadien-Kautschuk, Chloroprenkautschuk, chloriertes Polyethylen, Chlorsulfonylpolyethylen, Ethylen-Propylen-Kautschuk, Acrylatkautschuk und/oder Epichlorhydrinkautschuk, insbesondere Polyvinylchlorid.

10. Verwendung der Estermischungen gemäß Anspruch 9, **dadurch gekennzeichnet, dass** die Kunststoffe auch Zusatzstoffe enthalten wie Stabilisatoren, Gleitmittel, Füllstoffe, Pigmente, Flammschutzmittel, Lichtstabilisatoren, Treibmittel, polymere Verarbeitungshilfsmittel, Schlagzähverbesserer, optische Aufheller, Antistatika und/oder Biostabilisatoren sowie Mischungen davon.

11. Verwendung der Estermischungen gemäß Anspruch 10, **dadurch gekennzeichnet, dass** die Kunststoffe auch weitere Weichmacher enthalten wie Monoalkylester der Benzoesäure, Benzoesäurediester von Mono-, Di-, Tri- oder Polyalkylenglycolen, Dialkylester aliphatischer Disäuren, Dialkylester aromatischer Disäuren, Trialkylester aromatischer Trisäuren, Phenylester von Alkansulfonsäuren, Alkyl- oder Arylester der Phosphorsäure, Polyester aus Dicarbonsäuren sowie Mischungen davon.
